# EUROPEAN PATENT APPLICATION

(11) **EP 4 414 959 A1**
(43) Date of publication of application: **14.08.2024**
(21) Application number: 23155609.3
(22) Date of filing: 08.02.2023
(51) Int. Cl.: G08B 21/04, A61B 5/11, G16H 50/30

(54) **COMPUTER-IMPLEMENTED METHOD FOR TRAINING OF A MACHINE LEARNING MODEL IN ACCIDENT EVENT ASSESSMENT**

(71) Applicant: Autoliv Development AB, 447 83 Vårgårda (SE)
(72) Inventor: Alkhoury, Ziad, 95800 Paris (FR); Ahmed, Jawwad, 447 37 Vårgårda (SE)
(74) Representative: Westpatent AB

(57) **Abstract**

The present disclosure relates to a computer-implemented method for training of machine learning models in person accident event assessment. The method comprises preparing (S200) training data, by obtaining and automatically acquiring (S210) sensor data generated from sensors (2, 3; 6, 7) at an initial group (200) of subjects (201a-201c), and dividing (S220) the group (200) of subjects (201a-201c) into sub-groups (200a-200h) associated with certain corresponding features that are unique for the subjects (201a-201c) in that sub-group (200a-200h). The method further comprises training (S400) an initial machine learning model for the subjects in the initial group (200) and training (S500) a plurality of machine learning sub-models for the subjects (201a-201c) in the corresponding sub-group (200a-200h), such that one machine learning sub-model for each sub-group (200a-200h) is obtained.

## Description

### DESCRIPTION OF THE DISCLOSURE

The present disclosure relates to an improved computer-implemented method for training of a machine learning model in accident event assessment.

Accidents cause human injury and injury-related deaths. Accident detection, accident predictions, accident prevention and accident protection are together with machine learning approaches of increased usefulness.

Accident events include falls which for example may occur for a person walking in a home environment or outside the home, for example due to environmental conditions such as slippery foot paths or even people which are vulnerable to falls due to some medical conditions or post medical treatment. Medical treatment related to falls, particularly hip related fractures, can incur major medical expenses. There has been a lot of work on detecting falls in real-time and then using that information to trigger an emergency notification system so as to notify a hospital or a close relative of a person involved in an accident event.

There is recently also work on person protective equipment which can provide active safety to the user in case a fall is detected such as triggering a portable airbag system to protect the person on the vulnerable body parts from the impact of the fall such as hips. The algorithms running on such wearable devices must operate under "hard real-time conditions" so as to trigger the active safety with enough lead time so that this safety system can be activated into fully protective state.

Accident events also occur when riding a bicycle, scooter, motorcycle and the like, for example due to skidding, slipping, losing control of vehicle, crashing with another vehicle or object etc. Falls related to a running vehicle are often more abrupt and require a faster handling than a fall occurring for a walking person that for example trips or slips.

In particular motorcycle (MC) accidents can result in severe injuries or can turn fatal, where part of the reason is the more exposed nature of the rider on the MC as compared to the driver in a car. Also, there is no restraint on the MC such as a seat belt as in a car. There are also other motion dynamics which makes the MC rider far more vulnerable in case of accident or a crash. MCs with integrated airbag arrangements is an emerging technology which allows to reduce the risk of severe injury or fatality in case of an MC accident, particularly in a frontal crash or slightly angled crash helping to resist the MC rider from being thrown out away the MC as a result of the crash.

This can be life-saving when used effectively. However, it requires a real-time airbag triggering system to detect a crash in a few milliseconds and then subsequent triggering of a signal to inflate the airbag. A similar technology can also be used for a vest with one or more airbags or which inflates itself, or a smart helmet with one or more airbags, which are adapted to provide protection to the MC rider that wears at least one of the vest and the helmet when the MC rider is thrown away from the MC after the crash. Such a helmet may also be equipped with one or more devices that can issue one or more types of warnings. Other protective pieces of garment are conceivable, such as for example belts.

However, there is still room for improvements in this area. In particular, it is desired to provide training data for data-driven machine learning algorithms, i.e. for data-driven machine learning computer programs, that is adapted for different types of persons, both regarding physical properties and regarding behavioral properties.

This is achieved by means of a computer-implemented method for training of machine learning models in person accident event assessment comprising accident event detection. The method comprises implementing the machine learning models and preparing training data. Preparing training data comprises obtaining and automatically acquiring sensor data generated from sensors used for collecting data from an initial group of subjects, and dividing the group of subjects into sub-groups, where each sub-group is associated with certain features that are unique for the subjects in that sub-group.

The method further comprises communicating information comprising the prepared training data for machine learning model training, training an initial machine learning model using the training data comprising the prepared training data in fall assessment for the subjects in the initial group, and training a plurality of machine learning sub-models using the training data comprising the prepared training data in fall assessment for the subjects in the corresponding sub-group. In this way one machine learning sub-model for each sub-group is obtained.

This means that an initial, universal, machine learning model is trained first, and then a plurality of machine learning sub-models are trained. Subjects in a certain sub-group can make use of a corresponding machine learning sub-model that is more suited to the characteristics and needs of the subjects in that sub-group than the initial machine learning model.

Should the machine learning sub-model not be available or unsuitable for some reason, it is always possible to revert to the initial machine learning model for a certain subject or group of subjects. Both the initial machine learning model and the machine learning sub-models can thus be used for deployment on-device or in the cloud to provide active safety or some sort of e-call functionality.

According to some aspects, the computer-implemented method further comprises preparing individual training data for individual subjects in the corresponding sub-group by obtaining and automatically acquiring sensor data generated from sensors used for collecting data. The sensor data are generated during doings of individual subjects in the corresponding sub-group, said doings comprising activities of daily living (ADLs), including real fall events, and/or simulated falls. The method further comprises training individual machine learning models for the individual subjects using the individual training data comprising the prepared training data in fall assessment for each of the subjects in the corresponding sub-group, and using certain features that are unique for the individual subjects in that sub-group.

This means that new training data can be collected from the subjects on an individual basis such that individual machine learning models can be fine-tuned to the subjects in question. The more customized variants of each individual machine learning model that is produced improves accuracy and speed of detection of dangerous situations such as falls or near-falls under different scenarios of daily living activities. They can even introduce more fine-grained features and/or newer features for those individual users as the severity of the falls and/or fall risk assessment under their typical daily routine activities.

According to some aspects, the certain features comprise at least one of
- type of accident event,
- environmental conditions
- subject age,
- subject height,
- subject weight,
- subject gait patterns,
- subject medical conditions,
- driving characteristics, and
- vehicle type.

This means that many types of data can be used for fine tuning of the individual machine learning models based on individual personal data for each subject.

According to some aspects, the sensors comprise at least one of motion sensors, 3D sensors, accelerometers, gyroscopes and/or cameras. In other words, many types of well-known sensors can be used for acquiring the required data.

According to some aspects, wherein the subjects are persons that walk, and where the accident events are fall events that are due to tripping and/or slipping. According to some further aspects, the subjects are two-wheeled vehicle riders, and where the accident events are due to skidding, slipping, losing control of vehicle, and/or crashing with another vehicle or object.

This means that the present disclosure is applicable to both persons that walk and persons that ride two-wheeled vehicles, and the different types of associated accidents.

According to some aspects, preparing training data comprises obtaining and automatically acquiring current, and past historical data, including geographical positioning information of the user from positioning systems and/or weather information.

This increases the possibility to perform personalization at an even more fine-grained level.

The present disclosure also relates to a computer programs, control units, and systems that are associated with the above advantages.

Accordingly, it is here appreciated that the computer-implemented methods, the computer programs and the computer readable mediums, all as described herein, and in accordance with the present disclosure, may be realized in hardware, such as, the control unit arrangements and the devices, all as described herein, as well as, in the systems, as described herein. The hardware such as the control unit arrangements all as described herein, as well as, the systems, as described herein, are then arranged to perform the computer-implemented methods, and the computer programs, whereby the same advantages and effects are obtained as discussed for the computer-implemented methods herein.

### BRIEF DESCRIPTION OF DRAWINGS

The present disclosure will now be described more in detail with reference to the appended drawings, where:
- Figure 1: schematically illustrates a walking user wearing sensors;
- Figure 2: schematically illustrates a user who rides a motor bike and wears sensors;
- Figure 3: schematically illustrates groups of subjects;
- Figure 4: schematically illustrates a control unit;
- Figure 5: shows an example computer program product; and
- Figure 6: shows a flowchart for methods according to the present disclosure; and
- Figure 7: shows a flowchart for methods according to examples.

### DETAILED DESCRIPTION

Aspects of the present disclosure will now be described more fully hereinafter with reference to the accompanying drawings. The different arrangements, devices, systems, computer programs and methods disclosed herein can, however, be realized in many different forms and should not be construed as being limited to the aspects set forth herein. Like numbers in the drawings refer to like elements throughout.

The terminology used herein is for describing aspects of the disclosure only and is not intended to limit the invention. As used herein, the singular forms "a", "an" and "the" are intended to include the plural forms as well, unless the context clearly indicates otherwise.

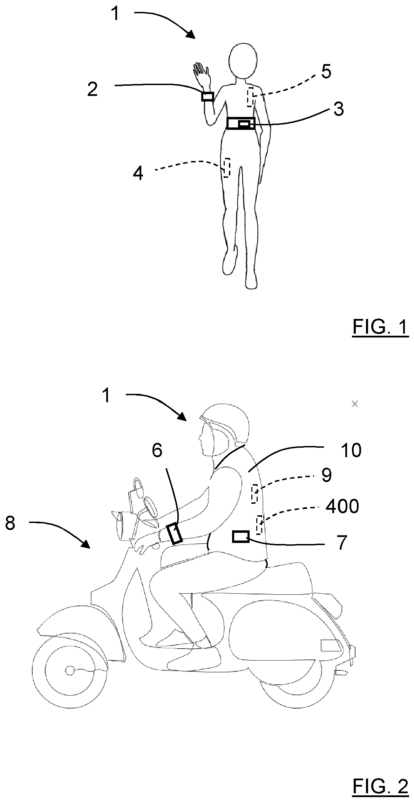

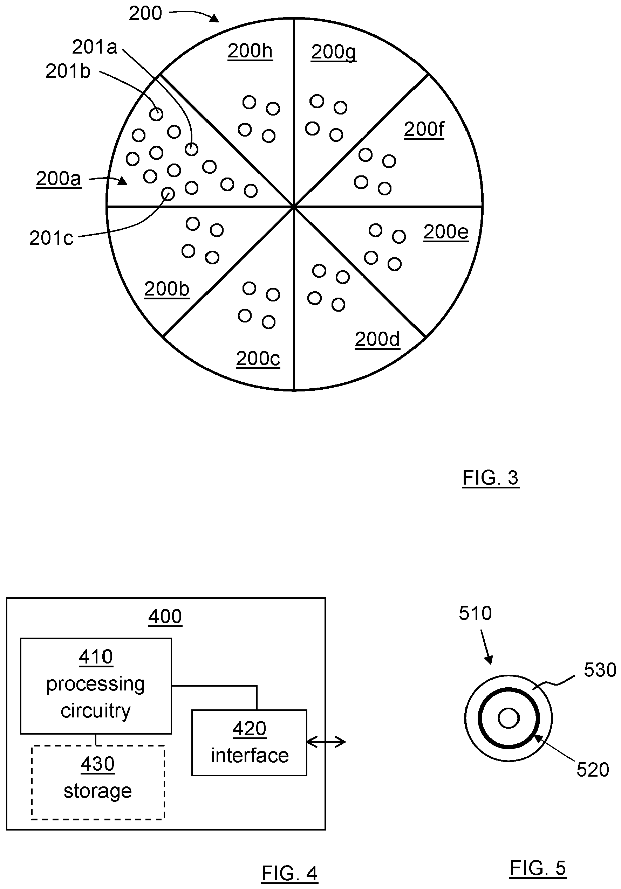

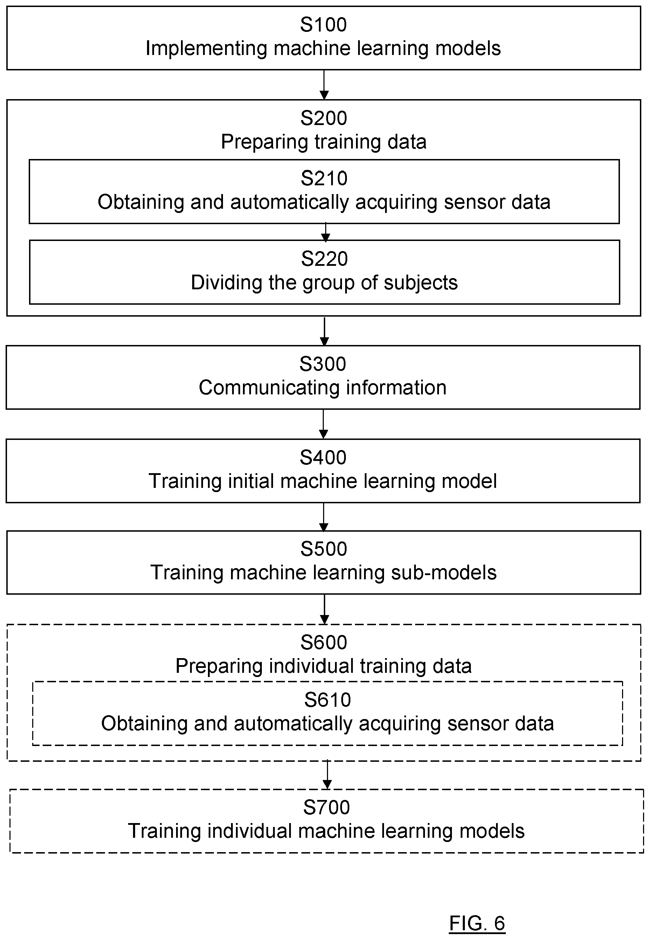

With reference to Figure 3 and Figure 6, the present disclosure relates to a computer-implemented method for training of a machine learning model in person accident event assessment comprising accident event detection. The method comprises implementing S100 the machine learning models and preparing S200 training data. With reference also to Figure 1 and Figure 2, preparing S200 training data in turn comprises obtaining and automatically acquiring S210 sensor data generated from sensors 2, 3; 6, 7 used for collecting data from an initial group 200 of subjects 201a-201c.

According to some aspects, accident event assessment also comprises accident event characterization and/or computation of accident event risk probability.

With reference to Figure 1, there is a subject 1 in the form of a user wearing at least one sensor device 2, 3, here one wrist sensor device 2 and one waist sensor device 3. In this case, the user 1 is walking.

With reference to Figure 2, there is a subject 1 in the form of a user 1 wearing at least one sensor device, here one wrist sensor device 6 and one vest sensor device 7 comprised in a protection garment such as a protection vest 10. In this case, the user 1 is riding a motor bike 8. The user 1 can wear sensors directly or indirectly attached to a garment that the user 1 is wearing.

The present disclosure is applicable for users that are walking or travelling on, or in, any kind of vehicle such as a bike, a motor bike 8, a car etc.

Sensor data are typically, but not exclusively, collected from, i.e. obtained from, sensors 2, 3, 6, 7, e.g. motion sensors, comprised in, e.g. mounted on, wearable devices, for example 3d sensors, such as accelerometers to measure the acceleration in 3 dimensions, as well as, gyroscope to measure the rotational speed in 3 dimensions from a person/a subject 1 i.e. trainer subject/s, and/or user 1, in accordance with the present disclosure, wearing such a wearable device 2, 3, 6, 7.

During experiments and other ways to collect data, i.e. during doings of the trainer subjects in accordance with present disclosure, with the wearable devices e.g. being 3d sensors, which suitably also are edge devices, data are generated by these 3d sensors at a specific frequency usually ranging from 25HZ-200HZ in the form of a multivariate time series which can be obtained, i.e. collected and buffered at the edge device before moving to an offline storage or uploaded to a Cloud device for further processing.

The obtained data may comprise data obtained from other sources than sensors. The obtained data may refer to current, and past historical, data. This may include geographical positioning information of the user from GPS system as well as weather information. According to some aspects, preparing S200 training data comprises obtaining and automatically acquiring S211 current, and past historical data, including geographical positioning information of the user from positioning systems and/or weather information. This increases the possibility to perform personalization at an even more fine-grained level.

According to some aspects, as illustrated in Figure 2, the user 1 wears means 9 for providing a position of the user 1 and communicating said position. Such means 9 can comprise any type of suitable positioning system, such as for example GPS or GNSS (Global Navigation Satellite Systems), and any type of wireless communication system. Said means can be comprised in a vest 10 or any suitable type of garment or wearable device.

The accident event detection, for example being accomplished by means of an accident event detection algorithm, relies on detecting patterns of motion which belong to an accident such as a fall, but real-world heterogeneity results in that fall patterns often are different depending on the different type of falls, environmental conditions but also on person age, height, weight, gait patterns and medical conditions, etc., as well as driving characteristics and vehicle type for a vehicle driver. It is therefore not possible to develop universal accident event detection which can work in an optimal way for each intended person. Personal traits and differences play a major role in heterogeneity which motivates the need to optimize the accident event detection based on personalization aspects.

Therefore, according to the present disclosure and with reference to Figure 3 and Figure 6, the method further comprises dividing S220 the initial group 200 of subjects 201a-201c into sub-groups 200a-200h, where each sub-group 200a-200h is associated with certain features that are unique for the subjects 201a-201c in that sub-group, and communicating S300 information comprising the prepared training data for machine learning model training. In Figure 3, for reasons of clarity, only a few subjects 201a-201c in a first sub-group 200a are indicated. Of course, there is a corresponding plurality of subjects in all sub-groups 200a-200h, only a few subjects are shown without indication in the other sub-groups 200b-200h, and there can be any suitable number of subjects 201a-201c in each sub-group 200a-200h. The certain features include certain characteristics.

The initial group 200 is here for reasons of clarity indicated as a circle, and the sub-groups 200a-200h are indicated as circle sectors that divide the circle into a number of parts, each part constituting a sub-group 200a-200h. In this example, there are eight equal part or sub-groups 200a-200h, but this is of course only an example, where the number of sub-groups may vary. The circular shape of the initial group 200 and the circle sector shapes that define the sub-groups are only chosen for illustrative purposes and should not be regarding as limiting in any way.

The method further comprises training S400 an initial machine learning model using the training data comprising the prepared training data in fall assessment for the subjects 201a-201c in the initial group 200, and training S500 a plurality of machine learning sub-models using the training data comprising the prepared training data in fall assessment for the subjects 201a-201c in the corresponding sub-group 200a-200h, such that one machine learning sub-model for each sub-group 200a-200h is obtained.

This means that an initial, universal, machine learning model is trained first, and then a plurality of machine learning sub-models are trained. This training can of course be performed in another order or at least partly in parallel.

It should be noted that some method steps may be executed in another order than as described above. For example, training S400 an initial machine learning model may be performed any time after obtaining and automatically acquiring S210 sensor data generated from sensors 2, 3; 6, 7 used for collecting data from an initial group 200 of subjects 201a-201c.

Then subjects 201a-201c in a certain sub-group 200a can make use of a corresponding machine learning sub-model that is more suited to the characteristics and needs of the subjects in that sub-group than the initial machine learning model. Should the machine learning sub-model not be available or unsuitable for some reason, it is always possible to revert to the initial machine learning model for a certain subject or group of subjects. Both the initial machine learning model and the machine learning sub-models can thus be used for deployment on-device or in the cloud to provide active safety or some sort of e-call functionality.

According to some aspects, initial data are collected from subjects 201a-201c falling naturally or collected in a controlled environment (i.e., fall data) as well as data from daily activities from the representative group of subjects such as elderly people from care homes may be doing various daily routines. This data can then be used to devise suitable data augmentations suitable for time series data from motion sensors then using this augmented data to develop an initial machine learning model using techniques such as contrastive learning.

It is furthermore desired to have a machine learning model that is even more suited for the subjects 201a-201c. Therefore, according to some aspects, the computer-implemented method further comprises preparing S600 individual training data for individual subjects 201a-201c in the corresponding sub-group 200a by obtaining and automatically acquiring S610 sensor data generated from sensors 2, 3; 6, 7 used for collecting data.

The sensor data are generated during doings of the individual subjects 201a-201c in the corresponding sub-group 200a-200h, said doings comprising activities of daily living (ADLs), including real fall events, and/or simulated falls, including falls or crashes involving an MC or similar vehicle, the subjects being drivers of such vehicles.

The method further comprises training S700 individual machine learning models for the individual subjects 201a-201c using the individual training data comprising the prepared training data in fall assessment for each of the subjects 201a-201c in the corresponding sub-group 200a-200h, and using certain features that are unique for the individual subjects 201a-201c in that sub-group 200a-200h. As mentioned above, the certain features include certain characteristics.

This means that new training data can be collected from the subjects 201a-201c on an individual basis such that individual machine learning models can be fine-tuned to the subjects 201a-201c in question. According to an example, this can be performed for certain individual subjects 201a-201c in a sub-group 201, for example premium customers according to the data collected from those specific users or users in premium group. In other words, not all subjects in a sub-group need not be subject to that individual machine learning models, but only certain ones.

The more customized variants of each individual machine learning model that is produced improves accuracy and speed of detection of dangerous situations such as falls or near-falls under different scenarios of daily living activities. They can even introduce more fine-grained features and/or newer features for those individual users as the severity of the falls and/or fall risk assessment under their typical daily routine activities.

Here, fine-grained data such as the personal physical profile attributes weight, BMI, age, list of daily activities, medical history are collected, and the subjects 201a-201c in questions are according to some aspects asked to perform a set of pre-defined activities for defined number of times.

Based on the user data analysis, subjects 201a-201c can be mapped to sub-groups 200a-200h previously defined and the respective machine learning sub-model can be picked and then form the basis for fine tuning of the individual machine learning models based on individual personal data for each subject.

Note that above defined individual machine learning models also can be updated over time as new data arrives, or subjects' sets of motion patterns change in significant manner since the last time the respective individual machine learning model was developed/updated.

When a fully personalized individual machine learning model is not yet available for a certain subject 201a-201c, that subject 201a-201c can make use of the respective machine learning sub-model based on relevance. If this is not possible, the individual can resort to make use of the initial machine learning model.

According to some aspects, the present disclosure thus relates to an approach on how to do the personalization at more fine-grained level. Starting from data for all the subjects available for training, sensor data similarity and meta data such as user height/age, weight, medical history etc. are used to create sub-groups, clusters, of data which are then used to train machine learning sub-models.

When a new subject enters the system, he or she will not have any personal data, for example related to ADL or falls as well as falls or crashes involving an MC or similar vehicle. The system will determine the most relevant sub-groups 200a-200h using meta data relevance, and use the associated machine learning sub-model for that user until a sufficient amount of individual data is collected such that an individual machine learning model can be trained for that subject.

It can take some time before a sufficient amount of individual data is retrieved, and during this time subject will still get moderate level of personalization due to the relevant machine learning sub-model being used. Only in case no relevant machine learning sub-model can be found for the new subject it's possible to resort to using the initial machine learning model.

All new data is collected for a new subject that has entered the system then will also be propagated to the respective sub-group 200a-200h to which the subject belongs. This means that new collected data for each subject also improves the performance for other subjects that have similar characteristics, being in the same sub-group 200a-200h. This data can also be propagated up in the hierarchy to a universal level to improve the initial machine learning model. In this manner, all shared data from the subjects are used, where the fully personal individual machine learning models put much more weight to the samples which belong to that specific subject to which the individual machine learning model belongs during training.

According to some aspects, the certain features comprise at least one of
- type of accident event,
- environmental conditions
- subject age,
- subject height,
- subject weight,
- subject gait patterns,
- subject medical conditions,
- driving characteristics, and
- vehicle type.

This means that many types of data can be used for fine tuning of the individual machine learning models based on individual personal data for each subject.

According to some aspects, the sensors 2, 3; 6, 7 comprise at least one of motion sensors, 3D sensors, accelerometers, gyroscopes and/or cameras.

In other words, many types of well-known sensors can be used for acquiring the required data.

According to some aspects, the subjects 1, 201a-201e are persons that walk, and where the accident events are fall events that are due to tripping and/or slipping.

According to some aspects, the subjects 1, 201a-201e are two-wheeled vehicle riders, and where the accident events are due to skidding, slipping, losing control of vehicle, and/or crashing with another vehicle or object.

This means that the present disclosure is applicable to both persons that walk and persons that ride two-wheeled vehicles, and the different types of associated accidents.

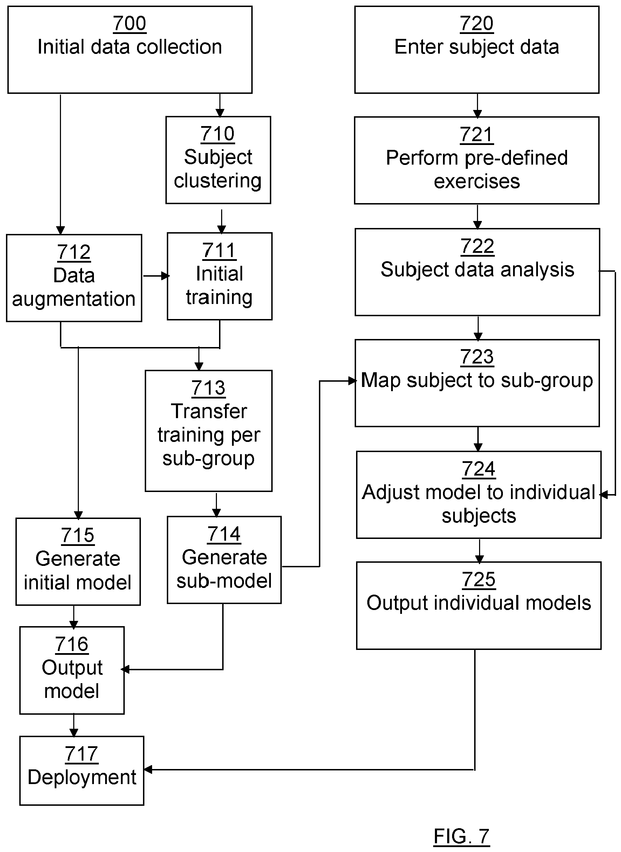

A non-limiting example where the person accident event assessment is a person fall event assessment will now be described with reference to Figure 3 and Figure 7.

After initial data collection 700 follows subject clustering 710 where the subjects are divided into sub-groups 200a-200h. Then an initial machine learning model is trained 711 parallel with data augmentation 712 using domain-specific techniques. The data augmentation 712 is related to the model training aspect of the models, which is indicated by an arrow to initial training 711. The result is transferred 713 to the sub-groups 200a-200h. Machine learning sub-models 714 are generated as well an initial machine learning model 715 which are output 716 and deployed 717.

For the individual machine learning models, subject data is entered 720 and the subjects perform pre-defined exercises 721 such as walking in a defined trajectory, sitting/standing up, laying down, climbing stairs, jogging (if possible), etc. This can be repeated, for example with a certain interval such as for example a number of months or weeks. As an alternative, or as a complement, automatic detections of exercises is also possible, using normal daily activities for adjusting the individual machine learning models.

Then subject data is analyzed 722, for example by means of a device or in an application. Then subject data is mapped 723 to a respective sub-group 200a-200h, using the machine learning sub-models 714. Then each individual machine learning model is adjusted 724 to fit individual subjects, and individual machine learning models are output 725 and deployed 717.

For the case where assessment is a two-wheel vehicle accident assessment, the steps discussed above are more or less the same with some modifications. In particular, the output model 716 is an initial vehicle accident detection machine learning model. The pre-defined exercises 721 can for example be pre-defined vehicle maneuvers such as a subject driving on different types of roads, in a straight line, at round abouts, stopping at traffic lights, rough driving etc.

Figure 4 schematically illustrates, in terms of a number of functional units, the components of the control unit 400 according to an embodiment.

Processing circuitry 410 is provided using any combination of one or more of a suitable central processing unit (CPU), multiprocessor, microcontroller, digital signal processor (DSP), dedicated hardware accelerator, etc., capable of executing software instructions stored in a computer program product, e.g. in the form of a storage medium 430. The processing circuitry 410 may further be provided as at least one application specific integrated circuit (ASIC), or field programmable gate array (FPGA) .

Particularly, the processing circuitry 410 is configured to cause the control unit 400 to perform a set of operations, or steps. These operations, or steps, were discussed above in connection to the various radar transceivers and methods. For example, the storage medium 430 may store the set of operations, and the processing circuitry 410 may be configured to retrieve the set of operations from the storage medium 430 to cause the control unit 400 to perform the set of operations. The set of operations may be provided as a set of executable instructions. Thus, the processing circuitry 410 is thereby arranged to execute methods and operations as herein disclosed.

The storage medium 430 may also comprise persistent storage, which, for example, can be any single one or combination of magnetic memory, optical memory, solid state memory or even remotely mounted memory.

The control unit 400 may further comprise a communications interface 420 for communications with at least one other unit. As such, the communications interface 420 may comprise one or more transmitters and receivers, comprising analogue and digital components and a suitable number of ports for wired or wireless communication.

The processing circuitry 410 is adapted to control the general operation of the control unit 400 e.g. by sending data and control signals to the external unit and the storage medium 430, by receiving data and reports from the external unit, and by retrieving data and instructions from the storage medium 430. Other components, as well as the related functionality, of the control unit 400 are omitted in order not to obscure the concepts presented herein.

The control unit 400 can be realized by one separate item or several items that together form a control unit arrangement. The control unit can at least be realized at a remote server, for example in a cloud service.

Figure 5 shows a computer program product 510 comprising computer executable instructions 520 arranged on a computer readable medium 530 to execute any of the methods disclosed herein.

According to some aspects, the present disclosure also relates to a device for person accident event assessment, for communication of information, wherein the information comprises information from the person accident event assessment, and for enabling the computer-implemented method for person accident event assessment, wherein the computer-implemented method for fall assessment is as described herein. The device comprises a machine learning model, sensors collecting data from users, means for obtaining data, means for processing data and means for communication of information. The device comprises the computer program, and/or the computer readable medium, both, as described herein, and for applying the computer-implemented method for person accident event assessment, and for communication of information, wherein the information comprises information from the person accident event assessment, as described herein.

According to some aspects, the device for person accident event assessment according to the present disclosure, as described herein, is disclosed, wherein the device is wearable by a user 1 and comprises sensor/s 2, 3, 6, 7 comprising accelerometer/s and/or gyroscope/s.

The present disclosure also relates to a system for training of a machine learning model in person accident event assessment, for communication of information, for enabling implementing the machine learning model as described herein, and for enabling the preparation of training data as described herein. Person accident event assessment comprises accident event detection.

The system comprises a machine learning model, trainer subjects 1, sensors collecting data from the trainer subjects, means for obtaining data, means for processing data and means for communication of information. The system utilizes the computer program 510 as described herein, and/or the computer readable medium 530 as described herein.

According to some aspects, the information comprises information from the person accident event assessment, as described herein, and/or the computer readable medium 530 comprising the computer program 510 product comprising computer readable instructions 520 for applying the computer-implemented method for person accident event assessment, and for communication of information, wherein the information comprises information from the person accident event assessment, as described herein.

According to some aspects, a system for person accident event assessment, and for communication of information, is disclosed. The information comprises information from the person accident event assessment, according to the present disclosure, as described herein, is disclosed. The system comprises one, or more, devices for person accident event assessment, and for communication of information, wherein the information comprises information from the person accident event assessment, as described herein.

## Claims

1. A computer-implemented method for training of machine learning models in person accident event assessment comprising accident event detection, where the method comprises
implementing (S100) the machine learning models, and
preparing (S200) training data,
where preparing (S200) training data comprises
obtaining and automatically acquiring (S210) sensor data generated from sensors (2, 3; 6, 7) used for collecting data from an initial group (200) of subjects (201a-201c); and
dividing (S220) the group (200) of subjects (201a-201c) into sub-groups (200a-200h), where each sub-group (200a-200h) is associated with certain features that are unique for the subjects (201a-201c) in that sub-group (200a-200h); where the computer-implemented method further comprises
communicating (S300) information comprising the prepared training data for machine learning model training;
training (S400) an initial machine learning model using the training data comprising the prepared training data in fall assessment for the subjects in the initial group (200);
training (S500) a plurality of machine learning sub-models using the training data comprising the prepared training data in fall assessment for the subjects (201a-201c) in the corresponding sub-group (200a-200h), such that one machine learning sub-model for each sub-group (200a-200h) is obtained.

2. The computer-implemented method according to claim 1, further comprising
preparing (S600) individual training data for individual subjects (201a-201c) in the corresponding sub-group (200a-200h) by
obtaining and automatically acquiring (S610) sensor data generated from sensors (2, 3; 6, 7) used for collecting data, where the sensor data are generated during doings of individual subjects (201a-201c) in the corresponding sub-group (200a-200h), said doings comprising activities of daily living, ADLs, including real fall events, and/or simulated falls; and
training (S700) individual machine learning models for the individual subjects (201a-201c) using the individual training data comprising the prepared training data in fall assessment for each of the subjects (201a-201c) in the corresponding sub-group (200a-200h), and using certain features that are unique for the individual subjects (201a-201c) in that sub-group (200a-200h) .

3. The computer-implemented method according to any one of the claims 1 or 2, wherein the certain features comprise at least one of
- type of accident event,
- environmental conditions
- subject age,
- subject height,
- subject weight,
- subject gait patterns,
- subject medical conditions,
- driving characteristics, and
- vehicle type.

4. The computer-implemented method according to any one of the previous claims, wherein the sensors (2, 3; 6, 7) comprise at least one of motion sensors, 3D sensors, accelerometers, gyroscopes and/or cameras.

5. The computer-implemented method according to any one of the previous claims, wherein the subjects (1; 201a-201c) are persons that walk, and where the accident events are fall events that are due to tripping and/or slipping.

6. The computer-implemented method according to any one of the claims 1-4, wherein the subjects (1; 201a-201c) are two-wheeled vehicle riders, and where the accident events are due to skidding, slipping, losing control of vehicle, and/or crashing with another vehicle or object.

7. The computer-implemented method according to any one of the previous claims, wherein preparing (S200) training data comprises obtaining and automatically acquiring (S211) current, and past historical data, including geographical positioning information of the user from positioning systems and/or weather information.

8. A computer program (510) comprising computer readable instructions (520) for applying the computer-implemented method, and/or the preparation of training data, according to any one of claims 1-7, and/or a computer readable medium (530) comprising said computer program (510).

9. A computer program (510) comprising computer readable instructions (520) for a machine learning model according to any one of claims 1-7, and/or a computer readable medium (530) comprising said computer program (510).

10. A control unit (400), for training of a machine learning model, adapted to control at least, the implementing the machine learning model according to any of claims 1-7, and/or the preparation of training data according to any one of claims 1-7.

11. A system for training of a machine learning model in person accident event assessment, for communication of information, for enabling implementing the machine learning model according to any one of claims 1-7, and for enabling the preparation of training data according to any one of claims 1-7;
wherein person accident event assessment comprises accident event detection;
where the system comprises a machine learning model, trainer subjects (1), sensors collecting data from the trainer subjects, means for obtaining data, means for processing data and means for communication of information;
wherein the system utilizes the computer program (510) according to claim 8 or 9, and/or the computer readable medium (530) according to claim 8 or 9.
